Europäisches Patentamt

European Patent Office

Office européen des brevets

(19)

(11) Veröffentlichungsnummer: 0 294 715
A2

(12) EUROPÄISCHE PATENTANMELDUNG

(21) Anmeldenummer: 88108878.5

(51) Int. Cl.⁴: B01D 53/36

(22) Anmeldetag: 03.06.88

(30) Priorität: 09.06.87 DE 3719174

(43) Veröffentlichungstag der Anmeldung:
14.12.88 Patentblatt 88/50

(84) Benannte Vertragsstaaten:
DE ES FR GB IT SE

(71) Anmelder: LEYBOLD AKTIENGESELLSCHAFT
Bonner Strasse 498
D-5000 Köln 51(DE)

(72) Erfinder: Voss, Günter, Dr.
Werschberg 60
D-5203 Much(DE)

(74) Vertreter: Leineweber, Jürgen, Dipl.-Phys.
Nagelschmiedshütte 8
D-5000 Köln 40(DE)

(54) Verfahren zur Überprüfung der Wirksamkeit eines Katalysators.

(57) Die Erfindung bezieht sich auf ein Verfahren zur Überprüfung der Wirksamkeit eines Katalysators; um diese schnell und exakt festzustellen, wird vorgeschlagen, daß - in Strömungsrichtung des Abgases -vor dem Katalysator (2) ein aus einem Stickoxid und einem Inertgas bestehendes Gasgemisch eingelassen wird, daß das aus dem Katalysator austretende Gas auf das Vorhandensein der Komponenten des eingelassenen Gasgemisches untersucht wird und daß zur Feststellung des Prüfungsergebnisses das Mischungsverhältnis der eingelassenen Gaskomponenten mit dem Mischungsverhältnis der austretenden Gaskomponenten verglichen wird.

FIG.1

EP 0 294 715 A2

## Verfahren zur Überprüfung der Wirksamkeit eines Katalysators

Die Erfindung bezieht sich auf ein Verfahren zur Überprüfung der Wirksamkeit eines Katalysators sowie ein Gerät zur Durchführung dieses Verfahrens.

Katalysatoren haben die Aufgabe, die in Motorabgasen vorhandenen Stickoxide abzubauen. Aus den verschiedensten Gründen kann die Funktion eines Katalysators beeinträchtigt sein. Neben Defekten an den Gasführungssystemen, Alterungserscheinungen oder dergleichen kann insbesondere die Verwendung von bleihaltigen Kraftstoffen die Wirksamkeit des Katalysators stark reduzieren. Meßverfahren, die eine einfache und - schnelle Feststellung der Wirksamkeit eines Katalysators im eingebauten Zustand erkennen lassen, sind nicht bekannt.

Der vorliegenden Erfindung liegt die Aufgabe zugrunde, ein Verfahren zur Überprüfung der Wirksamkeit eines Katalysators sowie ein für die Durchführung dieses Verfahrens geeignetes Gerät vorzuschlagen. Die Überprüfung der Wirksamkeit des Katalysators soll einfach, schnell und exakt möglich sein.

Erfindungsgemäß wird diese Aufgabe dadurch gelöst, daß -in Strömungsrichtung des Abgases - vor dem Katalysator ein aus einem Stickoxid und einem Inertgas bestehendes Gasgemisch eingelassen wird, daß das aus dem Katalysator austretende Gas auf das Vorhandensein der Komponenten des eingelassenen Gasgemisches untersucht wird und daß zur Feststellung des Prüfungsergebnisses das Mischungsverhältnis der eingelassenen Gaskomponenten mit dem Mischungsverhältnis der austretenden Gaskomponenten verglichen wird.

Ein Verfahren dieser Art erlaubt insbesondere dann, wenn zur Analyse der Gase ein Massenspektrometer verwendet wird, schnelle und exakte Messungen.

Als Komponenten des Meßgases kommen zweckmäßigerweise Stickstoffmonoxid und Argon infrage. Stickstoffmonoxid ist zu 90 % der beim Normalbetrieb der Brennkraftmaschine vom Katalysator abzubauende Stickoxid-Anteil. Außerdem ist die Wirksamkeit eines Katalysators für ein bestimmtes Stickoxid direkt vergleichbar mit der Wirksamkeit dieses Katalysators für andere Stickoxide.

Das Inertgas Argon durchströmt den Katalysator ohne jede Beeinflussung oder Veränderung. Seine Masse (40) liegt nicht weit von der Masse des Stickstoffmonoxids (30) entfernt, was meßtechnische Vorteile hat.

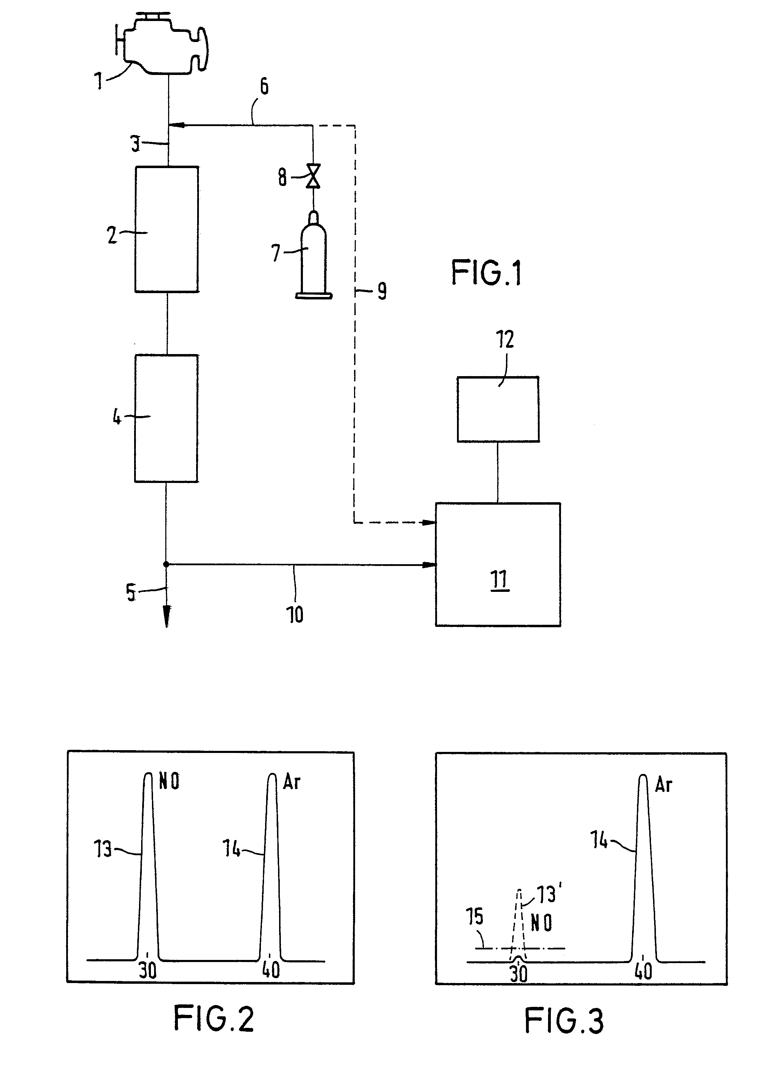

Weitere Vorteile und Einzelheiten der Erfindung sollen anhand der Figuren 1 bis 3 erläutert werden.

Die in Fig. 1 dargestellte Abgasanlage des Motors 1 umfaßt den Katalysator 2, denAbgasleitungsabschnitt 3, welche den Motor 1 mit dem Katalysator 2 verbindet, sowie die Auspuffanlage mit Auspuff 4 und Abgasendrohr 5.

Um die Wirksamkeit des Katalysators 2 zu überprüfen, wird über die Meßgas-Zuführungsleitung 6 in die Abgasleitung 3 das Meßgasgemisch eingelassen, das zum Beispiel zu jeweils 50 % aus Stickstoffmonoxid und Argon besteht. Die Abgasleitung 3 ist üblicherweise mit einem Meßanschluß ausgerüstet, der zum Beispiel der Temperaturmessung der Abgase dient. In Abhängigkeit der gemessenen Temperaturwerte kann zum Beispiel ein Dreiwege-Katalysator geregelt werden.

Das zuzuführende Gasgemisch befindet sich im Vorratsbehälter 7, aus dem es nach dem Öffnen des Ventils 8 durch die Zuführungsleitung 6 ausströmt. Die Zuführung des Meßgases hat in einer solchen Menge zu erfolgen, daß die ausströmende Stickoxid-Menge 5 % der insgesamt durch die Abgasanlage strömenden Gasmenge nicht übersteigt, da sonst die Kapazität des Katalysators überschritten wird. Ist das Mischungsverhältnis der Meßgas-Komponenten nicht bekannt oder soll es unmittelbar vor der Überprüfung der Wirksamkeit des Katalysators 2 nochmals kontrolliert werden, dann kann ein Anteil des Meßgasgemisches über die gestrichelt dargestellte Leitung 9 dem Massenspektrometer 11 direkt zugeführt werden.

Fig. 2 zeigt die Linien 13 und 14 des Meßgemisches bei der Verwendung von Stickstoffmonoxid (Masse 30) und Argon (Masse 40) unter der Voraussetzung, daß das Mischungsverhältnis 1 : 1 beträgt.

Das eingelassene Meßgasgemisch strömt in den Katalysator ein, in dem - falls der Katalysator voll funktionsfähig ist - das Stickoxid zu mehr als 90 % abgebaut wird. Das Argon durchströmt den Katalysator, ohne einen Einfluß zu erfahren. Ist der Katalysator teilweise oder völlig unwirksam, dann wird auch das Stickoxid im Katalysator in dem entsprechenden Maße nicht mehr abgebaut.

Zur Feststellung der Funktion des Katalysators wird das zum Beispiel aus dem Endrohr 5 austretende Gas mit Hilfe des Massenspektrometers 11 analysiert. Das geschieht mit Hilfe einer beheizten Kapillaren, die in das Endrohr 5 eingeführt wird und an die sich die zum Massenspektrometer 11 führende Leitung 10 anschließt.

Während des Einlassens des Meßgasgemisches in den Leitungsabschnitt 3 wird in jedem Fall Argon festzustellen sein, da es den Katalysator unbeeinflußt durchströmt. Figur 3 zeigt die Argonlinie 14, die mit gleicher Höhe eingezeichnet ist wie in Figur 2.

Je nach Wirksamkeit des zu untersuchenden Katalysators wird eine Stickoxid-Linie 13' unterschiedlicher Höhe erscheinen. Ist der Katalysator voll funktionstüchtig, dann wird die NO-Linie eine sehr kleine Höhe haben. Hat die NO-Linie die gleiche Höhe wie die Argon-Linie, dann ist der Katalysator völlig unwirksam.

Um eine Störung der Messung durch die Abgase zu verhindern, ist es zweckmäßig, unmittelbar vor dem Einlaß des Meßgases den durch die Abgase selbst verursachten Untergrund zu messen, zu speichern und vom nach dem Meßgaseinlaß festgestellten Ergebnis abzuziehen.

Dem Massenspektrometer 11 ist das Anzeigegerät 12 zugeordnet. Es kann entweder die Wirksamkeit des untersuchten Katalysators anhand einer Figur demonstrieren, die der Figur 3 entspricht. Bleibt die NO-Linie unter der strichpunktiert eingezeichneten Horizontallinie 15, dann ist der Katalysator in Ordnung. Übersteigt die Höhe der NO-Linie diesen Grenzwert, dann ist eine Schadstoffarmut oder -freiheit nicht mehr gegeben. Eine andere Möglichkeit besteht in der Anzeige von Zahlenwerten, die dem Konzentrationsverhältnis Argon zu NO - oder umgekehrt - entsprechen.

Das Gerät zur Durchführung des erfindungsgemäßen Verfahrens umfaßt das Massenspektrometer 11 mit den erforderlichen Versorgungseinrichtungen (Elektronik, Vakuumpumpen), das Anzeigegerät 12 und die notwendigen Gaszuführungsleitungen 9 und/oder 10. Auch der Vorratsbehälter 7 für das Meßgasgemisch kann in das erfindungsgemäße Gerät integriert sein.

Das erfindungsgemäße Verfahren ist schnell und exakt durchführbar und kommt deshalb mit extrem kleinen Gasmengen, vor allem Stickoxidmengen, aus. Es ist ohne weiteres möglich, das beispielhaft angegebene Mischungsverhältnis des Meßgasgemisches derart zu wählen, daß das Inertgas stark überwiegt, da die Gasanalyse mit einem Massenspektrometer extrem empfindlich ist. Die das erfindungsgemäße Gerät bedienenden Personen sind nicht gefährdet.

## Ansprüche

1. Verfahren zur Überprüfung der Wirksamkeit eines Katalysators, dadurch gekennzeichnet, daß - in Strömungsrichtung des Abgases - vor dem Katalysator (2) ein aus einem Stickoxid und einem Inertgas bestehendes Gasgemisch eingelassen wird, daß das aus dem Katalysator austretende Gas auf das Vorhandensein der Komponenten des eingelassenen Gasgemisches untersucht wird und daß zur Feststellung des Prüfungsergebnisses das Mischverhältnis der eingelassenen Gaskomponenten mit dem Mischungsverhältnis der austretenden Gaskomponenten verglichen wird.

2. Verfahren nach Anspruch 1, dadurch gekennzeichnet, daß es bei in Betrieb befindlicher Brennkraftmaschine (1) durchgeführt wird und daß der infolge des Betriebs vorhandene Untergrund vor der Vergleichsmessung abgezogen wird.

3. Verfahren nach Anspruch 1 oder 2, dadurch gekennzeichnet, daß die Messung der Gaskonzentrationen mit Hilfe eines Massenspektrometers (11) durchgeführt wird.

4 . Verfahren nach einem der vorhergehenden Ansprüche, dadurch gekennzeichnet, daß als Stickoxid Stickstoffmonoxid (NO) verwendet wird.

5. Verfahren nach einem der vorhergehenden Ansprüche, dadurch gekennzeichnet, daß als Inertgas Argon verwendet wird.

6. Verfahren nach einem der vorhergehenden Ansprüche, dadurch gekennzeichnet, daß das Mischungsverhältnis von Stickoxid zu Inertgas im Ausgangsgasgemisch 1 : 1 beträgt.

7. Gerät zur Durchführung des Verfahrens nach einem der vorhergehenden Ansprüche, dadurch gekennzeichnet, daß es ein Massenspektrometer (11) mit den zugehörigen Versorgungseinrichtungen sowie ein Anzeigegerät (12) umfaßt.

8. Gerät nach Anspruch 7, dadurch gekennzeichnet, daß es eine Gaszuführungsleitung (10) in einer beheizten Eintritts-Kapillaren aufweist.

9. Gerät nach Anspruch 7 oder 8, dadurch gekennzeichnet, daß es den Vorratsbehälter (7) für das Meßgasgemisch umfaßt.

FIG.1

FIG.2

FIG.3